# EUROPEAN PATENT SPECIFICATION

(11) **EP 1 441 689 B1**
(45) Date of publication and mention of the grant of the patent: **12.04.2006**
(21) Application number: 02787567.3
(22) Date of filing: 05.11.2002
(51) Int. Cl.: A61K 8/21, A61K 8/24, A61K 8/27, A61K 8/43, A61K 8/97, A61K 8/11, A61Q 11/00

(54) **SOLID ORAL COMPOSITIONS**
FESTE MUNDPFLEGEZUSAMMENSETZUNGEN
COMPOSITIONS ORALES SOLIDES

(30) Priority: 06.11.2001 IT MI20010232
(43) Date of publication of application: 04.08.2004
(73) Proprietor: Perfetti Van Melle S.p.A., 20020 Lainate (Milan) (IT)
(72) Inventor: COLLE, Roberto, I-20020 Lainate (IT); SALMOIRAGHI, Guglielmo, I-20020 Lainate (IT); BARRICA, Andrea, I-20020 Lainate (IT)
(74) Representative: Minoja, Fabrizio
(86) International application number: PCT/EP2002/012330
(87) International publication number: WO 2003/039504

(56) References cited:
- WO-A-00/30465
- WO-A-01/17494
- WO-A-01/56399
- US-A- 5 000 944
- US-A- 5 958 380
- "PDR for Herbicidal Medicines" * page 369 * * page 757 *
- GERALD KOWITZ ET AL: 'The effects of tartar-control toothpaste on the oral soft tissues' ORAL SURG ORAL MED ORAL PATHOL 1990, pages 529 - 536
- DELATTRE V.F.: 'Factors contributing to adverse soft tissue reactions due to the use of tartar control toothpastes' PERIODONTOL 1999, pages 803 - 807

## Description

The present invention relates to oral anti-tartar and anti-plaque compositions, useful as adjuvants in odontostomatological hygiene.

### BACKGROUND TO THE INVENTION

The problem of dental plaque and tartar formation has long been studied, and agents which can be used to combat and delay such formation are being actively researched.

The mechanisms that cause tartar deposits are well known; these deposits are constituted by calcium phosphate crystals which precipitate in the extracellular matrix of bacterial plaque. The pathogenetic role of tartar in periodontal diseases such as pyorrhoea, periodontitis, gingivitis and correlated disorders is equally well known.

Various substances have proved effective in reducing or preventing tartar formation and deposits on the teeth, including soluble pyrophosphates and polyphosphates, zinc salts, fluorides, diphosphonates, antibacterial agents such as triclosan, and abrasive agents such as silica or alumina. These substances, combined with one another in various ways, are included in the composition of most anti-tartar toothpastes now commercially available. The clinical efficacy of these toothpastes has been examined in numerous studies, reviewed in J. Clin. Dent. Vol IV(3), 71-81, 1993.

The most common toothpastes contain soluble polyphosphates associated with fluorides and silica, and possibly with polymers that possess bioadhesive properties, as described, for example, in US 4327977, US 4889713, US 5017362, US 5139769, US 4921693 and EP 492997.

Similar compositions, with the addition of antibacterial agents such as triclosan, are described, for example, in GB 2200551. In addition to toothpastes, chewing gums and candies with a similar composition have been developed.

The efficacy of these toothpastes, which has been the object of numerous studies (J. Clin. Dent. Vol. X(3), 99-102, 1999; Oral Surg. Oral Med. Oral Pathol., Vol 70(4), 529-536, 1990; J. Clin. Dent. Vol. IX(4), 101-104, 1998), is due to inhibition of calcium phosphate precipitation by the polyphosphates that complex the calcium ions in the saliva, to the abrasive action of silica, to the reinforcing effect of fluorides on the tooth enamel and to the action of bioadhesive polymers, where used, which protects the mucosae and causes slow release of the other ingredients.

Candies and chewing gum designed as adjuvants in dental hygiene and oral hygiene in general, which have properties that can be described as anti-tartar, anti-decay, whitening and/or refreshing, are becoming increasingly popular. The main advantage of these forms of administration is that they can be used freely and conveniently during the day in any place and on any occasion, in addition to that fact that the release of the active elements (functional ingredients) is slower and more regular than in the case of an ordinary toothpaste.

### DESCRIPTION OF THE INVENTION

The present invention relates to oral formulations in a solid form, preferably in the form of chewing gum, whose efficacy is superior to that of similar known formulations.

The compositions of the invention contain effective amounts of:
a. polyphosphates, preferably a mixture of alkali metal pyrophosphates and triphosphates;
b. an abrasive agent (preferably hydrated silica);
c. a source of fluoride ions;
d. extracts of vegetable origin;
e. optionally antibacterial or disinfectant agents.

In addition to the active ingredients referred to above, the compositions of the invention will contain excipients suitable to define the final form of administration.

Thus, for example, a chewing gum formulation will require a suitable base consisting of gum base, sweeteners, polyalcohols such as xylitol, sorbitol and mannitol, flavourings, dyes, softeners, plasticisers, stabilisers, thickeners, etc.

The polyphosphates used in the compositions may be alkali metal pyrophosphates (diphosphates), hexametaphosphates or tripolyphosphates or mixtures thereof. A mixture of disodium diacid diphosphate and pentasodium or pentapotassium triphosphate is particularly preferred. It has been proved that a toothpaste containing this mixture provides more marked reduction of tartar than a toothpaste containing pyrophosphates not associated with triphosphates (J. Clin. Dent. Vol. IX(4), 101-104, 1998).

Broadly, the polyphosphates amount to between 0.5 and 5% by weight on the total composition of the invention.

The function of the abrasive agent is to increase the plaque-removing action already possessed by ordinary chewing gum. It may be formed by hydrated silica (in a suitable form), calcium carbonate (in a suitable form) or talc, either individually or in a mixture thereof. These abrasives may also be present totally or partly, individually or combined with one another, in encapsulated form, in particular encapsulated in calcium alginate. Chewing gum containing microgranules of hydrated silica encapsulated in calcium alginate has proved more effective in removing plaque than a chewing gum with the same formulation but without microgranules (Doc. Os 06.2001 779-781). According to the present invention, the encapsulated microgranules may contain dyes, flavourings, functional ingredients and herb extracts. This abrasive agent is usually present in percentages of between 0.5 and 7% by weight.

Suitable sources of fluoride ions include sodium fluoride, potassium fluoride, ammonium fluoride, sodium monofluoro-phosphate and other known non-toxic salts containing fluorine, in concentrations which provide fluoride percentages of between 0.005 and 0.2% by weight.

The vegetable extracts which may be present in the compositions of the invention are selected from extracts of *Centella asiatica, Malva sylvestris, Melaleuca alternifolia, Commiphora abyssinica* (myrrh), *Krameria triandra* (rhatany), *Acacia catechu, Medicago sativa* (alfalfa), resins of the genus *Styrax,* such as *Styrax benzoin* (benzoin), *Matricaria recutita* (camomile), *Echinacea purpurea* (echinacea) and *Croton lechleri* (dragon's blood). Extracts of these plants, whose activity has been known for some time, are commercially available.

The combination of these extracts gives the formulations anti-inflammatory/decongestant, emollient, wound-healing, antiseptic and astringent properties. These properties are desirable in at least two respects in the ambit of the present invention:
firstly, to assist and reinforce the reduction in diseases of the oral mucosa caused by the reduction in tartar, and
secondly, to control and prevent contact stomatitis similar to that manifested with the use of toothpastes, known as "toothpaste stomatitis", in particularly predisposed persons.

In the formulations according to the invention, these extracts may be encapsulated in alginate together with the abrasive agent.

Said extracts may be added to the formulations in percentages of between 0.01 and 2% by weight.

The formulations of the invention can be prepared by conventional techniques, by adding and mixing the various ingredients to the gum base in the case of chewing gum, which may then undergo coating operations in accordance with equally conventional techniques.

The formulations of the invention may include disinfectant or antibacterial agents such as triclosan, zinc salts or zinc oxide, either alone or combined with one another, in concentrations of between 0.1 and 5% by weight. These agents are designed to combat the formation of bacterial plaque, which leads to tartar deposits.

The formulations of the invention may also include decorative crystals, preferably consisting of gum arabic and dyes deposited on the surface of the product with a purely aesthetic function.

Daily use of the chewing gum according to the invention reduces tartar deposits and has other beneficial effects on the condition of the oral and gingival mucosa.

The example below illustrates the invention in greater detail.

### EXAMPLE

**Coated chewing gum weighing 1.4 g with vegetable extracts.**

| Percentage composition (by weight) | |
|---|---|
| Ingredient | % |
| Gum base | 25.5 |
| Xylitol | 23.5 |
| Sorbitol | 23.2 |
| Mannitol | 16 |
| Flavouring | 1.8 |
| Silicon dioxide | 3 |
| Gum arabic | 1 |
| Glycerin | 1 |
| Disodium diacid diphosphate | 1 |
| Pentasodium triphosphate | 1 |
| Mallow, myrrh, centella, melaleuca, rhatany and cutch extracts | 0.05 |
| Maltitol syrup | 0.93 |
| Titanium dioxide (E171) | 0.7 |
| Quick Coat | 0.6 |
| Aspartame | 0.6 |
| Acesulfame | 0.05 |
| Carnauba wax | 0.05 |
| Potassium fluoride | 0.02 |
| Total | 100 |

## Claims

1. Oral formulations in the form of chewing gums, comprising:
a. polyphosphates;
b. an abrasive agent;
c. a source of fluoride ions;
d. extracts of vegetable origin;
e. optionally antibacterial or disinfectant agents
wherein the extracts have anti-inflammatory, wound-healing, antihemorrhagic, soothing, emollient, decongestant and antiseptic properties and are selected from extracts of *Centella asiatica, Malva sylvestris, Melaleuca alternifolia, Commiphora abyssinica* (myrrh), *Krameria triandra* (rhatany), *Acacia catechu, Medicago sativa* (alfalfa), resins of the genus *Styrax,* such as *Styrax benzoin* (benzoin), *Matricaria recutita* (camomile), *Echinacea purpurea* (echinacea) and *Croton lechleri* (dragon's blood).

2. Formulations as claimed in claim 1, wherein the polyphosphates are selected from tripolyphosphates, pyrophosphates or mixtures thereof.

3. Formulations as claimed in claim 2, comprising a mixture of alkali metal pyrophosphates and tripolyphosphates.

4. Formulations as claimed in any one of the preceding claims, further containing excipients selected from gum base, sweeteners, polyalcohols, flavourings, dyes, softeners, plasticisers, stabilisers and thickeners.

5. Formulations as claimed in the preceding claims, wherein the abrasive agent is hydrated silica, calcium carbonate and talc, either individually or in a mixture thereof.

6. Formulations as claimed in the preceding claims, containing 0.5 to 5% by weight of polyphosphates, 0.5 to 7% by weight of an abrasive agent (possibly wholly or partly encapsulated), a source of fluorine ions able to guarantee a fluoride intake of 0.005 to 0.2%, and 0.01 to 2% by weight of plant extracts.

7. Formulations as claimed in the preceding claims, comprising disinfectant and/or antibacterial agents selected from triclosan, zinc oxide and zinc salts, either alone or in combination with one another, in concentrations of between 0.1 % and 5%.

## Revendications

1. Formulations orales sous la forme de gommes à mâcher, comprenant :
a. des polyphosphates
b. un agent abrasif ;
c. une source d'ions fluorure ;
d. des extraits d'origine végétale ;
e. facultativement, des agents antibactériens ou désinfectants
dans lesquelles les extraits ont des propriétés anti-inflammatoires, cicatrisantes, antihémorragiques, lénifiantes, émollientes, décongestionnantes et antiseptiques et sont choisis parmi les extraits de *Centella asiatica, Malva sylvestris, Melaleuca altemifolia, Commiphora abyssinica* (myrrhe), *Krameria triandra* (ratanhia), *Acacia catechu, Medicago sativa* (luzerne), résines du genre *Styrax,* telles que de *Styrax benjoin* (benjoin), *Matricaria recutita* (camomille), *Echinacea purpurea* (échinacée) et *Croton lechleri* (sang dragonnier).

2. Formulations telles que revendiquées dans la revendication 1, dans lesquelles les polyphosphates sont choisis parmi des tripolyphosphates, des pyrophosphates ou leurs mélanges.

3. Formulations telles que revendiquées dans la revendication 2, comprenant un mélange de pyrophosphates et tripolyphosphates de métaux alcalins.

4. Formulations telles que revendiquées dans l'une quelconque des revendications précédentes, contenant de plus des excipients choisis parmi une gomme base; des édulcorants, des polyols, des aromatisants, des colorants, des ramollissants, des plastifiants, des stabilisants et des épaississants.

5. Formulations telles que revendiquées les revendications précédentes, dans lesquelles l'agent abrasif est la silice hydratée, le carbonate de calcium et le talc, soit individuellement, soit en mélange entre eux.

6. Formulations telles que revendiquées dans les revendications précédentes, contenant 0,5 à 5 % en poids de polyphosphates, 0,5 à 7 % en poids d'un agent abrasif (éventuellement totalement ou partiellement encapsulé), une source d'ions fluor capable d'assurer un apport de fluorure de 0,005 à 0,2 %, et 0,01 à 2 % en poids d'extraits végétaux.

7. Formulations telles que revendiquées dans les revendications précédentes, comprenant des agents désinfectants et/ou antibactériens choisis parmi le triclosan, l'oxyde de zinc et les sels de zinc, soit individuellement, soit en association entre eux, à des concentrations comprises entre 0,1 % et 5 %.

## Patentansprüche

1. Orale Zubereitungen in Form von Kaugummis, umfassend:
a. Polyphosphate;
b. ein abrasives Mittel;
c. eine Quelle von Fluoridionen,
d. Extrakte pflanzlichen Ursprungs;
e. gegebenenfalls antibakterielle oder desinfizierende Mittel,
wobei die Extrakte entzündungshemmende, wundheilende, antihämorrhagische, beruhigende, weichmachende, abschwellende und antiseptische Eigenschaften haben und ausgewählt sind aus Extrakten von *Centella asiatica, Malva sylvestris, Melaleuca alternifolia, Commiphora abyssinica* (Myrrhe), *Krameria triandra* (Rhatania), *Acacia catechu, Medicago sativa* (Alfalfa), Harze der Gattung *Styrax,* wie *Styrax bezoin* (Benzoin), *Matricaria recutita* (Kamille), *Echinacea purpurea* (Echinacea) und *Croton lechleri* (Drachenblut).

2. Zubereitungen nach Anspruch 1, wobei die Polyphosphate ausgewählt sind aus Tripolyphosphaten, Pyrophosphaten oder Gemischen davon.

3. Zubereitungen nach Anspruch 2, umfassend ein Gemisch aus Alkalimetallpyrophosphaten und Tripolyphosphaten.

4. Zubereitungen nach einem der vorstehenden Ansprüche, zusätzlich enthaltend Hilfsmittel, ausgewählt aus Gummi-Basis, Süßungsmittel, Polyalkoholen, Aromastoffen, Farbstoffen, Weichmachern, Plastifizierungsmitteln, Stabilisatoren, und Verdickungsmitteln.

5. Zubereitungen nach einem der vorstehenden Ansprüche, wobei das abrasive Mittel hydratisiertes Silica, Calciumcarbonat, Talkum, entweder einzeln oder in einem Gemisch davon, ist.

6. Zubereitungen nach einem der vorstehenden Ansprüche, 0,5-5 Gew.-% Polyphosphate, 0,5-7 Gew.-% eines abrasiven Mittels (ggf. vollständig oder teilweise verkapselt), eine Quelle für Fluoridionen, die in der Lage ist eine Fluoridaufnahme von 0,005 bis 0,2 % sicherzustellen, und 0,01-2 Gew.-% Pflanzenextrakte enthaltend.

7. Zubereitungen nach einem der vorstehenden Ansprüche, desinfizierende und/oder bakterielle Mittel umfassend, ausgewählt aus Triclosan, Zinkoxid und Zinksalzen, entweder alleine oder in Kombination miteinander, in Konzentrationen zwischen 0,1 % und 5 %.
